# EUROPEAN PATENT SPECIFICATION

(11) **EP 2 251 387 B1**
(45) Date of publication and mention of the grant of the patent: **29.02.2012**
(21) Application number: 09160247.4
(22) Date of filing: 14.05.2009
(51) Int. Cl.: C09B 35/031, C09D 11/00, D06P 3/14, D06P 3/24, C09B 29/36

(54) **Acidic azo dyes**
Saure Azofarbstoffe
Colorants acides azoïques

(43) Date of publication of application: 17.11.2010
(73) Proprietor: Clariant International Ltd., 4132 Muttenz 1 (CH)
(72) Inventor: Nusser, Rainer Dr., 79395 Neuenburg (DE); Geiger, Ulrich Dr., 79395 Neuenburg/Rhein (DE); Hasemann, Ludwig Dr., 79379 Muellheim-Niederweiler (DE)
(74) Representative: Hütter, Klaus

(56) References cited:
- EP-A- 0 185 620
- WO-A-92/14791
- GB-A- 1 272 043
- GB-A- 1 458 295

## Description

The invention relates to novel acid dyes, a process for their preparation and their use for dyeing organic substrates.

Acid dyes are known. However, there is still a need for acid dyes with improved properties. GB 1 272 043, EP 0 185 620, GB 1 458 295 and WO 92/14791 disclose relevant mono-azo dyes.

The invention provides compounds of the general formula (I) 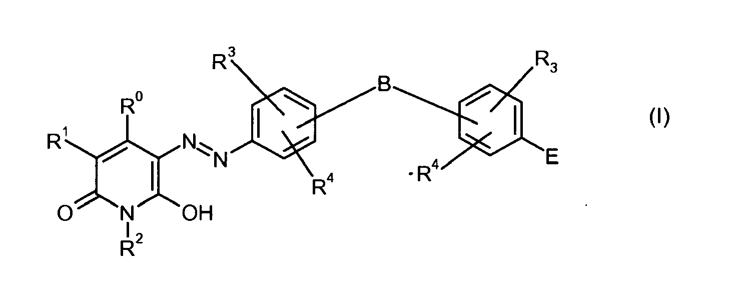
- E: signifies NH₂ or OH
- R⁰: signifies a substituted C₁ to C₄ alkyl group or an unsubstituted C₁ to C₄ alkyl group,
- R¹: signifies H, a substituted C₁ to C₄ alkyl group or an unsubstituted C₁ to C₄ alkyl group, a sulpho group, -CO-NH₂, -CO-NH-(C₁ to C₄ alkyl) or CN,
- R²: signifies H, a substituted C₁ to C₄ alkyl group or an unsubstituted C₁ to C₄ alkyl group,
- R³: signifies H, a sulpho group, a substituted C₁ to C₄ alkyl group or an unsubstituted C₁ to C₄ alkyl group, a substituted C₁ to C₄ alkoxy group or an unsubstituted C₁ to C₄ alkoxy group,
- R⁴: signifies H, a substituted C₁ to C₄ alkyl group or an unsubstituted C₁ to C₄ alkyl group, a substituted C₁ to C₄ alkoxy group or an unsubstituted C₁ to C₄ alkoxy group,
- B: a group with the formula -SO₂-, -NH-CO-NH-, -CR⁵R⁶-, wherein
- R⁵: signifies H, substituted C₁ to C₉ alkyl group or an unsubstituted C₁ to C₉ alkyl group,
- R⁶: signifies H, a substituted C₁ to C₉ alkyl group or an unsubstituted C₁ to C₉ alkyl group, an unsubstituted aryl group or a substituted aryl group or R⁵ and R⁶ form together a five or six membered cyclo aliphatic ring, wherein the
five or six membered rings are substituted by a C₁ to C₄ alkyl group or the five or six membered rings are not further substituted.

By preference, the sum of carbon atoms of R⁵ and R⁶ together is at least 4 carbon atoms, more preferred R⁵ and R⁶ have together at least 5 carbon atoms. Even more preferred, the sum of carbon atoms of R⁵ and R⁶ together is 5 or 6 or 7 or 8 or 9 carbon atoms.

The preferred compounds of formula (I) bear at least one anionic substituent, preferably 1 or 2 or 3 anionic substituents, of which 2 anionic substituents are very particularly preferred.

The at least one anionic substituent in the compounds of formula (I) are located by preference in one of the substituents R¹ and/or R³, more preferred, the at least one anionic substituent is located in one of the substituents R². Located by preference in one of the substituents may also mean that this substituent is the anionic group.

Preferred anionic substituents are carboxyl and/or sulpho groups, and sulpho groups are particularly preferred.

The preferred substituents of the substituted C₁ to C₄ alkyl groups are selected from the following substituents -OH, -O(C₁ to C₄ - Alkyl), -SO₃H, -COOH, -NH(C₁ to C₄ - Alkyl). The more preferred substituents of the substituted C₁ to C₄ alkyl groups are selected from the following substituents -OH, -O(C₁ to C₄ - Alkyl), -SO₃H, -COOH, -NH(C₁ to C₄ - Alkyl). The alkyl groups groups are branched or linear. The most preferred alkyl groups are methyl, ethyl, propyl, iso-proply, butyl, iso-butyl (2-Methylpropyl), pentyl, iso-pentyl (3-Methylbutyl, hexyl, heptyl, octyl, or nonyl

The preferred substituents of the substituted C₁ to C₄ alkoxy group are selected from the following substituents -OH, -O(C₁ to C₄ - Alkyl), -SO₃H, -COOH, -NH(C₁ to C₄ - Alkyl). The alkoxy groups are branched or linear.

Preferred substituents of the substituted aryl groups are selected from the following substituents -OH, -O(C₁ to C₄ - Alkyl), -SO₃H, substituted C₁ to C₄ alkyl groups, unsubstituted alkyl groups, a substituted C₁ to C₄ alkoxy group and an unsubstituted C₁ to C₄ alkoxy group.

In the preferred embodiment E signifies -NH₂.

In preferred compounds of the general formula (I)
- E: signifies NH₂ or OH
- R⁰: signifies an unsubstituted C₁ to C₂ alkyl group,
- R¹: signifies a substituted C₁ to C₂ alkyl group or an unsubstituted C₁ to C₂ alkyl group, a sulpho group, -CO-NH₂, -CO-NH-(C₁ to C₂ alkyl) or CN,
- R²: signifies a substituted C₁ to C₃ alkyl group or an unsubstituted C₁ to C₃ alkyl group,
- R³: signifies H, a sulpho group, an unsubstituted C₁ to C₂ alkyl group, a an unsubstituted C₁ to C₂ alkoxy group,
- R⁴: signifies H, an unsubstituted C₁ to C₂ alkyl group, a an unsubstituted C₁ to C₂ alkoxy group,
- B: a group with the formula -SO₂-, -CR⁵R⁶-, wherein
- R⁵: signifies H, substituted C₁ to C₉ alkyl group or an unsubstituted C₁ to C₉ alkyl group,
- R⁶: signifies a substituted C₁ to C₉ alkyl group or an unsubstituted C₁ to C₉ alkyl group, an unsubstituted aryl group or a substituted aryl group or R⁵ and R⁶ form together a five or six membered cyclo aliphatic ring, wherein the five or six membered rings are substituted by a C₁ to C₄ alkyl group or the five or six membered rings are not further substituted.

In even more preferred compounds of the general formula (I)
- E: signifies NH₂ or OH
- R⁰: signifies a methyl group,
- R¹: signifies -CH₂-SO₃H, -CONH₂ or -CN,
- R²: signifies a ethyl group, -CH₂-CH₂-CH₂-NH-CH₃, -CH₂-CH₂-COOH, or - CH₂-CH₂-CH₂-O-CH₂-CH₂-CH₂-CH₃
- R³: signifies H, methyl, methoxy or a sulpho group
- R⁴: signifies H, methyl or a methoxy group
- B: a group with the formula -SO₂- or -CR⁵R⁶-, wherein

- R⁵: signifies H, methyl or a ethyl group,
- R⁶: signifies an unsubstituted C₁ to C₄ alkyl group, a unsubstituted aryl group or a substituted aryl group or R⁵ and R⁶ form together a six membered cyclo aliphatic ring, wherein the six membered rings are not further substituted.

In the most preferred compounds of the general formula (I)
- E: signifies NH₂ or OH
- R⁰: signifies a methyl group,
- R¹: signifies -CH₂-SO₃H, -CONH₂ or -CN preferably a -CH₂-SO₃H group,
- R²: signifies a ethyl group, -CH₂-CH₂-CH₂-NH-CH₃, -CH₂-CH₂-COOH, or - CH₂-CH₂-CH₂-O-CH₂-CH₂-CH₂-CH₃, preferably a ethyl group,
- R³: signifies H, methyl, methoxy or a sulpho group, preferably H, methyl,
- R⁴: signifies H, methyl or a methoxy group preferably H,
- B: a group with the formula -SO₂- or -CR⁵R⁶-, wherein
- R⁵: signifies H,
- R⁶: signifies a unsubstituted aryl group or a substituted aryl group, preferably a unsubstituted aryl group wherein the aryl group is phenyl group.

The invention also provides a process for preparing compounds of the formula (I). The present invention's compounds of the formula (I) can be prepared under conventional conditions in conventional processes.

In these processes, both the amine functions or the amine function of compounds of the formula (II) 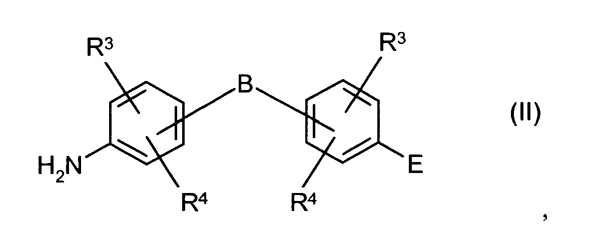 which are known from the literature are conventionally diazotized and coupled onto totally one equivalents of a compound of the formula (III) 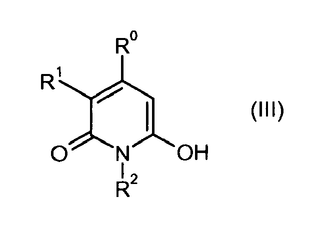 where the substituents are each as defined above.

In these processes, the particular compound of formula (II) is cooled to 0-10°C or preferably to 0-5°C and diazotized by adding nitrosylsulphuric acid or sodium nitrite. Afterwards, the diazotized or bis-diazotized diamine is allowed to react with the compound (III), preferably in aqueous solution.

The dyes of the formula (I) can be isolated from the reaction medium by conventional processes, for example by salting out with an alkali metal salt, filtering and drying, if appropriate under reduced pressure and at elevated temperature.

Depending on the reaction and/or isolation conditions, the dyes of the formula (I) can be obtained as free acid, as salt or as mixed salt which contains for example one or more cations selected from alkali metal ions, for example the sodium ion, or an ammonium ion or alkylammonium cation, for example mono-, di- or trimethyl- or -ethylammonium cations. The dye can be converted by conventional techniques from the free acid into a salt or into a mixed salt or vice versa or from one salt form into another. If desired, the dyes can be further purified by diafiltration, in which case unwanted salts and synthesis by-products are separated from the crude anionic dye.

The removal of unwanted salts and synthesis by-products and partial removal of water from the crude dye solution is carried out by means of a semipermeable membrane by applying a pressure whereby the dye is obtained without the unwanted salts and synthesis by-products as a solution and if necessary as a solid body in a conventional manner.

The dyes of the formula (I) and their salts are particularly suitable for dyeing or printing fibrous material consisting of natural or synthetic polyamides in yellow to greenish yellow shades. The dyes of the formula (I) and their salts are suitable for producing Inkjet printing inks and for using these Inkjet printing inks to print fibrous material which consists of natural or synthetic polyamides or cellulose (paper for example).

The invention accordingly provides from another aspect for the use of the dyes of the formula (I), their salts and mixtures for dyeing and/or printing fibrous materials consisting of natural or synthetic polyamides. A further aspect is the production of Inkjet printing inks and their use for printing fibrous materials consisting of natural or synthetic polyamides.

Dyeing is carried out as per known processes, see for example the dyeing processes described in Ullmanns Encyklopädie der technischen Chemie, 4th Edition, 1982, Volume 22, pages 658-673 or in the book by M. Peter and H.K. Rouette, Grundlagen der Textilveredlung, 13th Edition, 1989, pages 535-556 and 566-574. Preference is given to dyeing in the exhaust process at a temperature of 30 to 140°C, more preferably 80 to 120°C and most preferably at a temperature of 80 to 100°C, and at a liquor ratio in the range from 3:1 1 to 40:1.

The substrate to be dyed can be present in the form of yam, woven fabric, loop-formingly knitted fabric or carpet for example. Fully fashioned dyeings are even permanently possible on delicate substrates, examples being lambswool, cashmere, alpaca and mohair. The dyes of the invention are particularly useful for dyeing fine-denier fibres (microfibres).

The dyes according to the present invention and their salts are highly compatible with known acid dyes. Accordingly, the dyes of the formula (I), their salts or mixtures can be used alone in a dyeing or printing process or else as a component in a combination shade dyeing or printing composition together with other acid dyes of the same class, i.e. with acid dyes possessing comparable dyeing properties, such as for example fastness properties and exhaustion rates from the dyebath onto the substrate. The dyes of the present invention can be used in particular together with certain other dyes having suitable chromophores. The ratio in which the dyes are present in a combination shade dyeing or printing composition is dictated by the hue to be obtained.

The novel dyes of the formula (I), as stated above, are very useful for dyeing natural and synthetic polyamides, i.e. wool, silk and all nylon types, on each of which dyeings having a high fastness level, especially good light fastness and good wet fastnesses (washing, alkaline perspiration) are obtained. The dyes of the formula (I) and their salts have a high rate of exhaustion. The ability of the dyes of the formula (I) and their salt to build up is likewise very good. On-tone dyeings on the identified substrates are of outstanding quality. All dyeings moreover have a constant hue under artificial light. Furthermore, the fastness to decating and boiling is good.

One decisive advantage of the novel dyes is that they are metal free and provide very level dyeings.

The compounds according to the invention can be used as an individual dye or else, owing to their good compatibility, as a combination element with other dyes of the same class having comparable dyeing properties, for example with regard to general fastnesses, exhaustion value, etc. The combination shade dyeings obtained have similar fastnesses to dyeings with the individual dye.

The invention's dyes of the formula (I) can also be used as yellow components in trichromatic dyeing or printing. Trichromatic dyeing or printing can utilize all customary and known dyeing and printing processes, such as for example the continuous process, exhaustion process, foam dyeing process and Ink-Jet process.

The composition of the individual dye components in the trichromatic dye mixture used in the process of the invention depends on the desired hue. A brown hue for example preferably utilizes 20 - 40% by weight of a yellow component, 40 - 60% by weight of the invention's orange or red component and 10 - 20% by weight of a blue component.

The yellow component, as described above, can consist of a single component or of a mixture of different orange individual components conforming to the formula (I). Preference is given to double and triple combinations.

Particularly preferred red and/or blue components are described in WO2002/46318 or WO99/51681 respectively.

The following examples further serve to illustrate the invention. In the Examples all parts and all percentages are by weight or volume, and the temperatures given are in degrees Celsius, unless indicated to the contrary.

### Example 1:

26.8 Parts (0.1 mol) of 1,1-bis-(4-aminophenyl)-2-ethyl-butane are tetrazotised according to known methods with 13.8 parts (0.2 mol) of sodium nitrite at 0-5°C in 200 parts of water and 60 parts of hydrochloric acid (ca. 30%). 24.7 parts (0.1 mol) of a compound of the formula 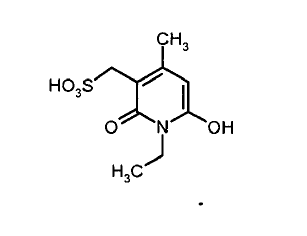 dissolved in 250 parts of water are added over 30 minutes to the ice cold tetrazotised solution. By the addition of 30% NaOH solution the pH is brought to 3-4.5 yielding a dyestuff of formula 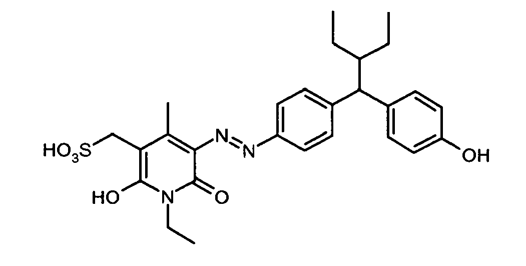 and the dyestuff is in solution. λ max = 447 nm.

The dyestuff can be isolated by concentration under vacuum or by precipitation in aceton/alcohol. The reaction mixture however can be used directly for dyeing without isolation the product. The dyestuff has surprisingly very high solubility in water and gives yellow dyeings with very good fastness properties.

### Example 2:

27.4 Parts (0.1 mol) of Bis-(4-aminophenyl)-phenylmethane are tetrazotised according to known methods with 13.8 parts (0.2 mol) of sodium nitrite at 0-5°C in 200 parts of water and 60 parts of hydrochloric acid (ca. 30%).
24.7 parts (0.1 mol) of a compound of the formula 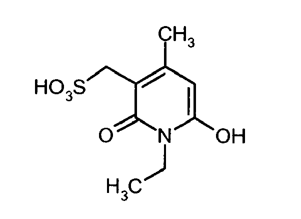 dissolved in 250 parts of water are added over 30 minutes to the ice cold tetrazotised solution. By the addition of 30% NaOH solution the pH is brought to 3-4.5 yielding a dyestuff of formula 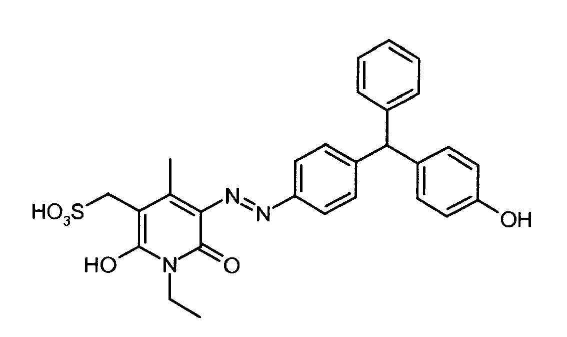 and the dyestuff is in solution. λ max = 459 nm.
The dyestuff can be isolated by concentration under vacuum or by precipitation in aceton/alcohol.

The reaction mixture however can be used directly for dyeing without isolation the product. The dyestuff has very high solubility in water and gives yellow dyeings with surprisingly very good fastnes properties.

### Examples 3-20:

The following compounds shown in the table 1 were synthesized according to the example 1 or 2 using the diamine 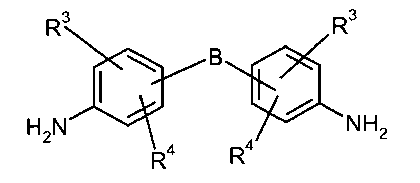 as diazo component and reacted with coupling component 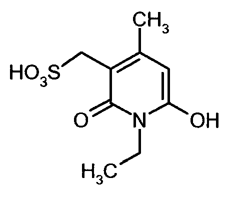 wherein a compound of the following formula was obtained: 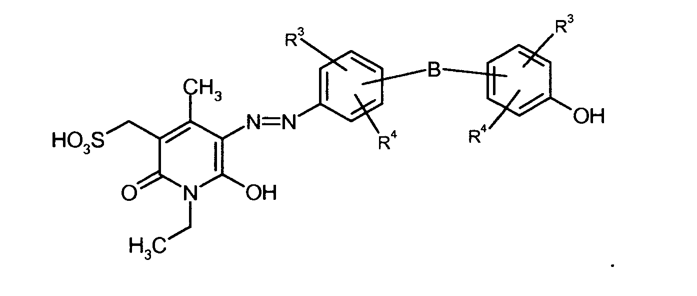 λ max (lambda max) is indicated in nm (nano meters; measured in 1% acetic acid solution). **Table 1:**

| **Example** | **Diamine** | **λₘₐₓ [nm]** |
|---|---|---|
| **3** | 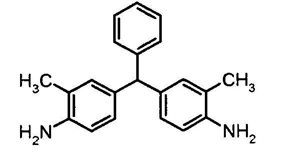 | 448 |
| **4** | 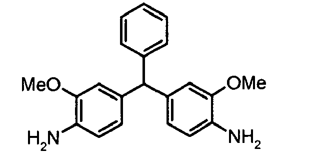 | 449 |
| **5** | 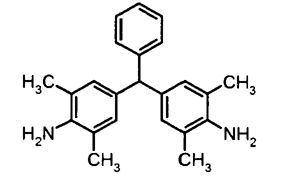 | 445 |
| **6** | 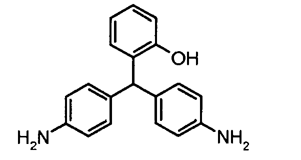 | 459 |
| **7** | 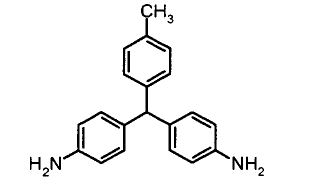 | 457 |
| **8** | 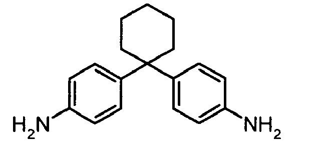 | 457 |
| **9** | 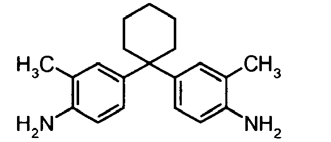 | 448 |
| **10** | 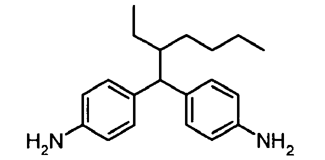 | 454 |
| **11** | 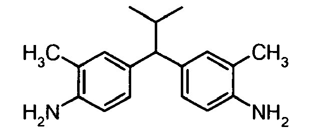 | 456 |
| **12** | 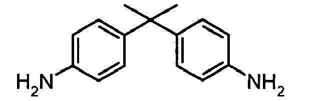 | 458 |
| **13** | 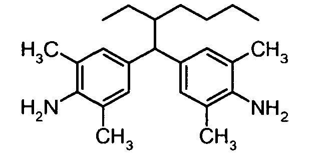 | 453 |

| | | |
|---|---|---|
| **14** | 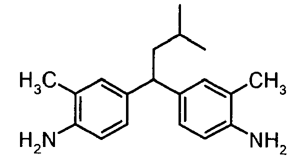 | 459 |
| **15** | 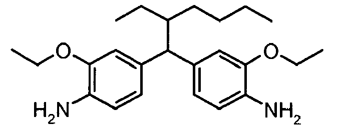 | 457 |
| **16** | 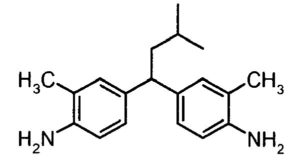 | 446 |
| **17** | 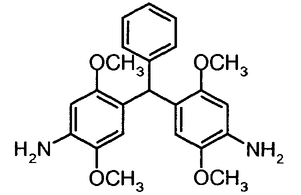 | 445 |
| **18** | 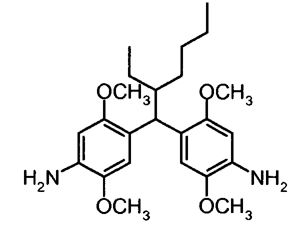 | 449 |
| **19** | 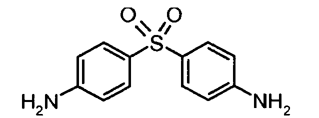 | 449 |
| **20** | 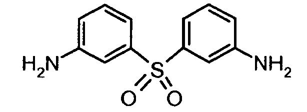 | 452 |

### Examples 21 - 26 :

The following compounds shown in the table 2 were synthesized according to the examples 1 or 2 using the diamine 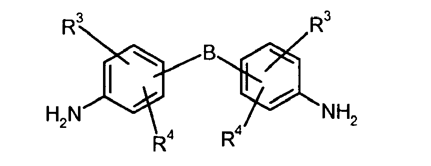 as diazo component and reacted with coupling component 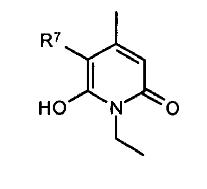 wherein a compound of the following formula was obtained: 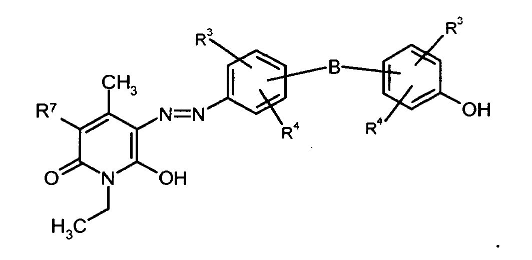 λ max (lambda max) is indicated in nm (nano meters; measured in 1% acetic acid solution).

**Table 2:**

| **Example** | **R⁷** | **Diamine** | **λₘₐₓ [nm]** |
|---|---|---|---|
| 21 | -CN | 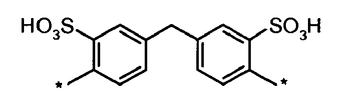 | 458 |
| 22 | Dto. | 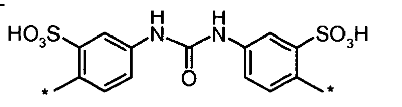 | 453 |
| 23 | -CONH₂ | Dto. | 459 |
| 24 | Dto. | 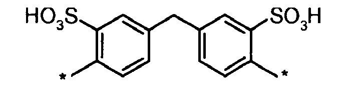 | 457 |
| 25 | -SO₃H | Dto. | 457 |
| 26 | Dto. | 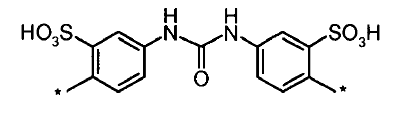 | 449 |

### Example 27:

27.4 Parts (0.1 mol) of 4, 4'-Diamino-diphenylsulphone are diazotised according to known methods with 6.9 parts (0.1 mol) of sodium nitrite at 0-5°C in 200 parts of water and 60 parts of hydrochloric acid (ca. 30%) .
24.7 parts (0.1 mol) of a compound of the formula 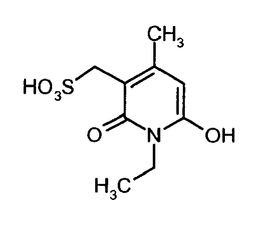 dissolved in 250 parts of water are added over 30 minutes to the ice cold diazotised solution. By the addition of 30% NaOH solution the pH is brought to 3-4.5 yielding a dyestuff of formula 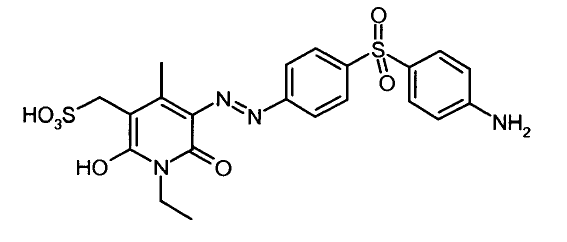 and the dyestuff is in solution. λ max = 462 nm.
The dyestuff can be isolated by concentration under vacuum or by precipitation in aceton/alcohol.

The reaction mixture however can be used directly for dyeing without isolation the product. The dyestuff has very high solubility in water and gives yellow dyeings with surprisingly very good fastnes properties.

### Examples 28-32:

The following compounds shown in the table 1 were synthesized according to the example 27 using the diamine 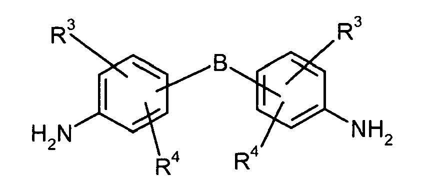 as diazo component and reacted with coupling component 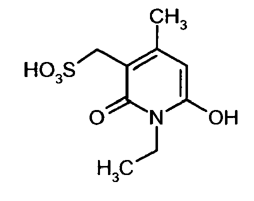 wherein a compound of the following formula was obtained: 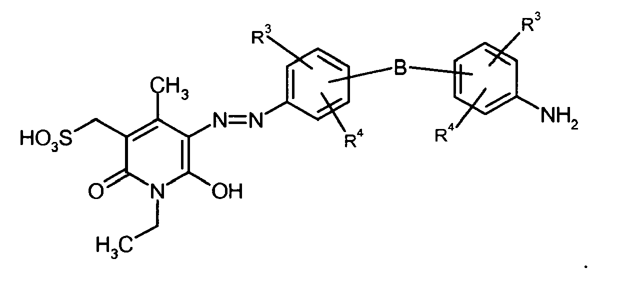 λ max (lambda max) is indicated in nm (nano meters; measured in 1% acetic acid solution).

**Table 3:**

| **Example** | **Diamine** | **λₘₐₓ [nm]** |
|---|---|---|
| **28** | 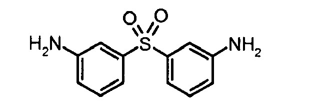 | 456 |
| **29** | 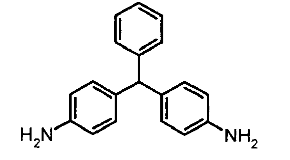 | 447 |
| **30** | 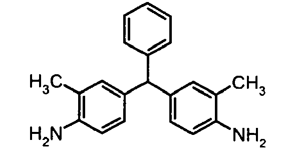 | 441 |
| **31** | 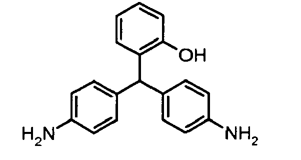 | 450 |
| **32** | 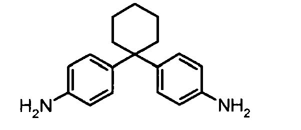 | 447 |

### USE EXAMPLE A

A dyebath at 40°C, consisting of 2000 parts of water, 1 part of a weakly cation-active levelling agent which is based on an ethoxylated aminopropyl fatty acid amide and which has affinity for dye, 0.25 part of the dye of Preparation Example 1 and adjusted to pH 5 with 1-2 parts of 40% acetic acid is entered with 100 parts of nylon-6 fabric. After 10 minutes at 40°C, the dyebath is heated to 98°C at a rate of 1°C per minute and then left at the boil for 45-60 minutes. Thereafter it is cooled down to 70°C over 15 minutes. The dyeing is removed from the bath, rinsed with hot and then with cold water and dried. The result obtained is a yellow polyamide dyeing possessing good light and wet fastnesses.

### USE EXAMPLE B

A dyebath at 40°C, consisting of 2000 parts of water, 1 part of a weakly cation-active levelling agent which is based on an ethoxylated aminopropyl fatty acid amide and which has affinity for dye, 0.3 part of the dye of Preparation Example 1 and adjusted to pH 5.5 with 1-2 parts of 40% acetic acid is entered with 100 parts of nylon-6,6 fabric. After 10 minutes at 40°C, the dyebath is heated to 120°C at a rate of 1.5°C per minute and then left at this temperature for 15-25 minutes. Thereafter it is cooled down to 70°C over 25 minutes. The dyeing is removed from the dyebath, rinsed with hot and then with cold water and dried. The result obtained is a yellow polyamide dyeing with good levelness and having good light and wet fastnesses.

### USE EXAMPLE C

A dyebath at 40°C, consisting of 4000 parts of water, 1 part of a weakly amphoteric levelling agent which is based on a sulphated, ethoxylated fatty acid amide and which has affinity for dye, 0.4 part of the dye of Preparation Example 1 and adjusted to pH 5 with 1-2 parts of 40% acetic acid is entered with 100 parts of wool fabric. After 10 minutes at 40°C, the dyebath is heated to boiling at a rate of 1°C per minute and then left at the boil for 40-60 minutes. Thereafter it is cooled down to 70°C over 20 minutes. The dyeing is removed from the bath, rinsed with hot and then with cold water and dried. The result obtained is a yellow wool dyeing possessing good light and wet fastnesses.

### USE EXAMPLE D

100 parts of a woven nylon-6 material are padded with a 50°C liquor consisting of

| | | |
|---|---|---|
| 40 | parts | of the dye of Preparation Example 1, |
| 100 | parts | of urea, |
| 20 | parts | of a nonionic solubilizer based on butyldiglycol, |
| 15-20 | parts | of acetic acid (to adjust the pH to 4), |
| 10 | parts | of a weakly cation-active levelling agent which is based on an ethoxylated aminopropyl fatty acid amide and has affinity for dye, and |
| 810-815 | parts | of water (to make up to 1000 parts of padding liquor). |

The material thus impregnated is rolled up and left to dwell in a steaming chamber under saturated steam conditions at 85-98°C for 3-6 hours for fixation. The dyeing is then rinsed with hot and cold water and dried. The result obtained is a yellow nylon dyeing having good levelness in the piece and good light and wet fastnesses.

### USE EXAMPLE E

A textile cut pile sheet material composed of nylon-6 and having a synthetic base fabric is padded with a liquor containing per 1000 parts

| | |
|---|---|
| 1 | part of dye of Preparation Example 1 |
| 4 | parts of a commercially available thickener based on carob flour ether |
| 2 | parts of a nonionic ethylene oxide adduct of a higher alkylphenol |
| 1 | part of 60% acetic acid. |

This is followed by printing with a paste which per 1000 parts contains the following components:

| | |
|---|---|
| 20 | parts of commercially available alkoxylated fatty alkylamine (displace product) |
| 20 | parts of a commercially available thickener based on carob flour ether. |

The print is fixed for 6 minutes in saturated steam at 100°C, rinsed and dried. The result obtained is a level-coloured cover material having a yellow and white pattern.

### USE EXAMPLE F

100 parts of a chrome-tanned and synthetically retanned shave-moist grain leather are dyed for 30 minutes in a bath of 300 parts of water and 2 parts of the dye of Preparation Example 1 at 55°C. After addition of 4 parts of a 60% emulsion of a sulphited fish oil, the leather is fatliquored for 45 minutes. It is then acidified with 8.5% formic acid and milled for 10 minutes (final pH in the bath 3.5-4.0). The leather is then rinsed, allowed to drip dry and finished as usual. The result obtained is a leather dyed in a level clear orange hue with good fastnesses.

Use Examples A to F can also be carried out with dyes 2 to 32 with similar results.

### USE EXAMPLE G

3 parts of the dye of Preparation Example 1 are dissolved in 82 parts of demineralized water and 15 parts of diethylene glycol at 60°C. Cooling down to room temperature gives an orange printing ink which is very highly suitable for ink jet printing on paper or polyamide and wool textiles.

Use Example G can also be carried out with dyes 2 to 26 with similar results.

### USE EXAMPLE H

A dyebath consisting of 1000 parts of water, 80 parts of calcined Glauber salt, 1 part of sodium nitrobenzene-3-sulphonate and 1 part of dye from Example 1 is heated to 80°C in the course of 10 minutes. Then, 100 parts of mercerized cotton are added. This is followed by dyeing at 80°C for 5 minutes and then heating to 95°C in the course of 15 minutes. After 10 minutes at 95°C, 3 parts of sodium carbonate are added, followed by a further 7 parts of sodium carbonate after 20 minutes and another 10 parts of sodium carbonate after 30 minutes at 95°C. Dyeing is subsequently continued at 95°C for 60 minutes. The dyed material is then removed from the dyebath and rinsed in running demineralized water for 3 minutes. This is followed by two washes for 10 minutes in 5000 parts of boiling demineralized water at a time and subsequent rinsing in running demineralized water at 60°C for 3 minutes and with cold tap water for one minute. Drying leaves a brilliant yellow cotton dyeing having good fastnesses.

### USE EXAMPLE I

0.2 part of the dye of Preparation Example 1 is dissolved in 100 parts of hot water and the solution is cooled down to room temperature. This solution is added to 100 parts of chemically bleached sulphite pulp beaten in 2000 parts of water in a Hollander. After 15 minutes of commixing the stuff is sized with resin size and aluminium sulphate in a conventional manner. Paper produced from this stuff has a yellow shade with good wet fastnesses.

Use Examples H and I can also be carried out with dyes 2 to 32 with similar results.

## Claims

1. Compounds of the general formula (I) 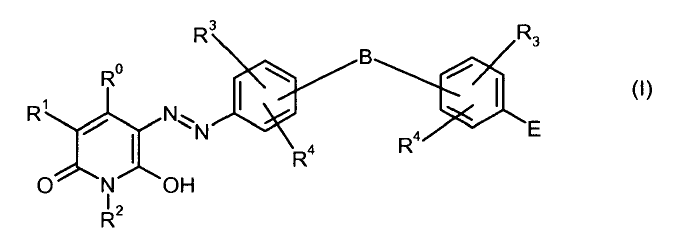 wherein
E signifies NH₂ or OH
R⁰ signifies a substituted C₁ to C₄ alkyl group or an unsubstituted C₁ to C₄ alkyl group,
R¹ signifies H, a substituted C₁ to C₄ alkyl group or an unsubstituted C₁ to C₄ alkyl group, a sulpho group, -CO-NH₂, -CO-NH-(C₁ to C₄ alkyl) or CN,
R² signifies H, a substituted C₁ to C₄ alkyl group or an unsubstituted C₁ to C₄ alkyl group,
R³ signifies H, a sulpho group, a substituted C₁ to C₄ alkyl group or an unsubstituted C₁ to C₄ alkyl group, a substituted C₁ to C₄ alkoxy group or an unsubstituted C₁ to C₄ alkoxy group,
R⁴ signifies H, a substituted C₁ to C₄ alkyl group or an unsubstituted C₁ to C₄ alkyl group, a substituted C₁ to C₄ alkoxy group or an unsubstituted C₁ to C₄ alkoxy group,
B a group with the formula -SO₂-, -NH-CO-NH-, -CR⁵R⁶-, wherein
R⁵ signifies H, substituted C₁ to C₉ alkyl group or an unsubstituted C₁ to C₉ alkyl group,
R⁶ signifies H, a substituted C₁ to C₉ alkyl group or an unsubstituted C₁ to C₉ alkyl group, an unsubstituted aryl group or a substituted aryl group or R⁵ and R⁶ form together a five or six membered cyclo aliphatic ring, wherein the five or six membered rings are substituted by a C₁, to C₄ alkyl group or the five or six membered rings are not further substituted.

2. Compounds according to claim 1 **characterized in that** the compounds of formula (I) bear at least one anionic substituent

3. Compounds according to claim 2 **characterized in that**
E signifies NH₂ or OH
R⁰ signifies an unsubstituted C₁ to C₂ alkyl group,
R¹ signifies a substituted C₁ to C₂ alkyl group or an unsubstituted C₁ to C₂ alkyl group, a sulpho group, -CO-NH₂, -CO-NH-(C₁ to C₂ alkyl) or CN,
R² signifies a substituted C₁ to C₃ alkyl group or an unsubstituted C₁ to C₃ alkyl group,
R³ signifies H, a sulpho group, an unsubstituted C₁ to C₂ alkyl group, a an unsubstituted C₁ to C₂ alkoxy group,
R⁴ signifies H, an unsubstituted C₁ to C₂ alkyl group, a an unsubstituted C₁ to C₂ alkoxy group,
B a group with the formula -SO₂-, -CR⁵R⁶-, wherein
R⁵ signifies H, substituted C₁ to C₉ alkyl group or an unsubstituted C₁ to C₉ alkyl group,
R⁶ signifies a substituted C₁ to C₉ alkyl group or an unsubstituted C₁ to C₉ alkyl group, an unsubstituted aryl group or a substituted aryl group or R⁵ and R⁶ form together a five or six membered cyclo aliphatic ring, wherein the five or six membered rings are substituted by a C₁ to C₄ alkyl group or the five or six membered rings are not further substituted.

4. Compounds according to claim 3 **characterized in that**
E signifies NH₂ or OH
R⁰ signifies a methyl group,
R¹ signifies -CH₂-SO₃H, -CONH₂ or -CN,
R² signifies a ethyl group, -CH₂-CH₂-CH₂-NH-CH₃, -CH₂-CH₂-COOH, or - CH₂-CH₂-CH₂-O-CH₂-CH₂-CH₂-CH₃
R³ signifies H, methyl, methoxy or a sulpho group
R⁴ signifies H, methyl or a methoxy group
B a group with the formula -SO₂- or -CR⁵R⁶-, wherein
R⁵ signifies H, methyl or a ethyl group,
R⁶ signifies an unsubstituted C₁ to C₄ alkyl group, a substituted aryl group or R⁵ and R⁶ form together a six membered cyclo aliphatic ring, wherein the six membered rings are not further substituted.

5. Process for preparing compounds of the formula (I) according to Claim 1, **characterized**
**in that** the amine functions of the compounds of the formula (II) 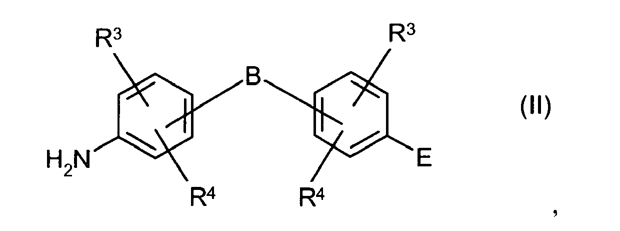 are diazotized and coupled onto totally one equivalents of a compound of the formula (III) 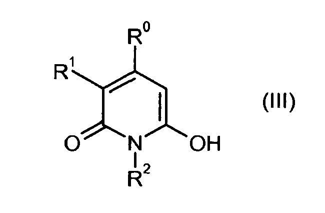 where the substituents are each as defined above.

6. Use of the compounds of the formula (I) according to Claim 1 for dyeing and/or printing organic substrates.

7. Use of compounds of formula (I) according to Claim 1 for dyeing and/or printing wool, silk and synthetic polyamides.

8. Use of compounds of formula (I) according to Claim 1 for preparing printing inks for the InkJet process.

## Patentansprüche

1. Verbindungen der allgemeinen Formel (I) 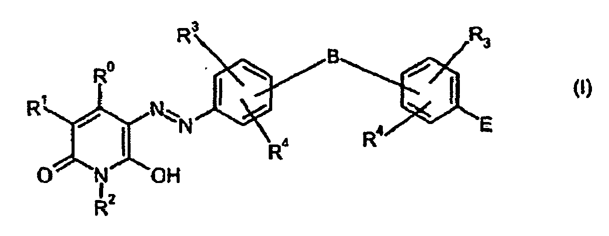 worin
E NH₂ oder OH,
R⁰ eine substituierte C₁- bis C₄-Alkylgruppe oder eine unsubstituierte C₁- bis C₄-Alkylgruppe,
R¹ H, eine substituierte C₁- bis C₄-Alkylgruppe oder eine unsubstituierte C₁- bis C₄-Alkyl- gruppe, eine Sulfogruppe, -CO-NH₂, -CO-NH- (C₁- bis C₄-Alkyl) oder CN,
R² H, eine substituierte C₁- bis C₄-Alkylgruppe oder eine unsubstituierte C₁- bis C₄-Alkyl- gruppe,
R³ H, eine Sulfogruppe, eine substituierte C₁- bis C₄-Alkylgruppe oder eine unsubstituierte C₁- bis C₄-Alkylgruppe, eine substituierte C₁- bis C₄-Alkoxygruppe oder eine unsubstituierte C₁- bis C₄-Alkoxygruppe,
R⁴ H, eine substituierte C₁- bis C₄-Alkylgruppe oder eine unsubstituierte C₁- bis C₄-Alkyl- gruppe, eine substituierte C₁- bis C₄- Alkoxygruppe oder eine unsubstituierte C₁- bis C₄-Alkoxygruppe,
B eine Gruppe der Formel -SO₂-, -NH-CO-NH-, -CR⁵R⁶-, worin
R⁵ H, eine substituierte C₁- bis C₉-Alkylgruppe oder eine unsubstituierte C₁- bis C₉-Alkyl- gruppe bedeutet,
R⁶ H, eine substituierte C₁- bis C₉-Alkylgruppe oder eine unsubstituierte C₁- bis C₉-Alkyl- gruppe, eine unsubstituierte Arylgruppe oder eine substituierte Arylgruppe bedeutet oder R⁵ und R⁶ zusammen einen fünf- oder sechsgliedri- gen cycloaliphatischen Ring bilden, wobei die fünf- oder sechsgliedrigen Ringe durch eine C₁- bis C₄-Alkylgruppe substituiert sind oder die fünf- oder sechsgliedrigen Ringe nicht weiter substituiert sind,
bedeuten.

2. Verbindungen gemäß Anspruch 1, **dadurch gekennzeichnet, dass** die Verbindungen der Formel (I) mindestens einen anionischen Substituenten tragen.

3. Verbindungen gemäß Anspruch 2, **dadurch gekennzeichnet, dass**
E NH₂ oder OH,
R⁰ eine unsubstituierte C₁- bis C₂-Alkylgruppe,
R¹ eine substituierte C₁- bis C₂-Alkylgruppe oder eine unsubstituierte C₁- bis C₂-Alkylgruppe, eine Sulfogruppe, -CO-NH₂, -CO-NH- (C₁- bis C₂- Alkyl) oder CN,
R² eine substituierte C₁- bis C₃-Alkylgruppe oder eine unsubstituierte C₁- bis C₃-Alkylgruppe,
R³ H, eine Sulfogruppe, eine unsubstituierte C₁- bis C₂-Alkylgruppe, eine unsubstituierte C₁- bis C₂-Alkoxygruppe,
R⁴ H, eine unsubstituierte C₁- bis C₂-Alkyl- gruppe, eine unsubstituierte C₁- bis C₂- Alkoxygruppe,
B eine Gruppe der Formel -SO₂-, -CR⁵R⁶-, worin
R⁵ H, eine substituierte C₁- bis C₉-Alkylgruppe oder eine unsubstituierte C₁- bis C₉-Alkyl- gruppe bedeutet,
R⁶ eine substituierte C₁- bis C₉-Alkylgruppe oder eine unsubstituierte C₁- bis C₉-Alkylgruppe, eine unsubstituierte Arylgruppe oder eine substituierte Arylgruppe bedeutet oder R⁵ und R⁶ zusammen einen fünf- oder sechsgliedrigen cycloaliphatischen Ring bilden, wobei die fünf- oder sechsgliedrigen Ringe durch eine C₁- bis Aralkylgruppe substituiert sind oder die fünf- oder sechsgliedrigen Ringe nicht weiter substituiert sind,
bedeuten.

4. Verbindungen gemäß Anspruch 3, **dadurch gekennzeichnet, dass**
E NH₂ oder OH,
R⁰ eine Methylgruppe,
R¹ -CH₂-SO₃H, -CONH₂ oder -CN,
R² eine Ethylgruppe, -CH₂-CH₂-CH₂-NH-CH₃, -CH₂- CH₂-COOH oder -CH₂CH₂-CH₂-O-CH₂CH₂CH₂CH_{3'}
R³ H, Methyl, Methoxy oder eine Sulfogruppe,
R⁴ H, Methyl oder eine Methoxygruppe,
B eine Gruppe der Formel -SO₂- oder -CR⁵R⁶-, worin
R⁵ H, Methyl oder eine Ethylgruppe bedeutet,
R⁶ eine unsubstituierte C₁- bis C₄-Alkylgruppe, eine unsubstituierte Arylgruppe oder eine substituierte Arylgruppe bedeutet oder R⁵ und R⁶ zusammen einen sechsgliedrigen cyclo- aliphatischen Ring bilden, wobei die sechs- gliedrigen Ringe nicht weiter substituiert sind,
bedeuten.

5. Verfahren zur Herstellung von Verbindungen der Formel (I) gemäß Anspruch 1, **dadurch gekennzeichnet, dass** man die Aminfunktionen der Verbindungen der Formel (II) 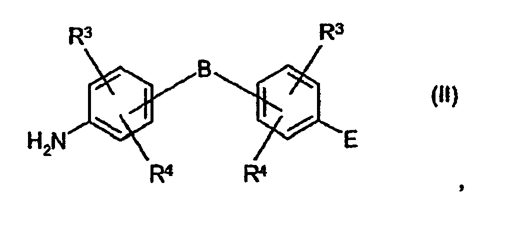 diazotiert und auf insgesamt ein Äquivalent einer Verbindung der Formel (III) 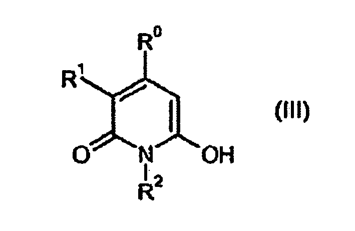 kuppelt, wobei die Substituenten jeweils die oben angegebenen Bedeutungen haben.

6. Verwendung der Verbindungen der Formel (I) gemäß Anspruch 1 zum Färben und/oder Bedrucken von organischen Substraten.

7. Verwendung von Verbindungen der Formel (I) gemäß Anspruch 1 zum Färben und/oder Bedrucken von Wolle, Seide und synthetischen Polyamiden.

8. Verwendung von Verbindungen der Formel (I) gemäß Anspruch 1 zur Herstellung von Drucktinten für das InkJet-Verfahren.

## Revendications

1. Composés représentés par la formule générale (I) 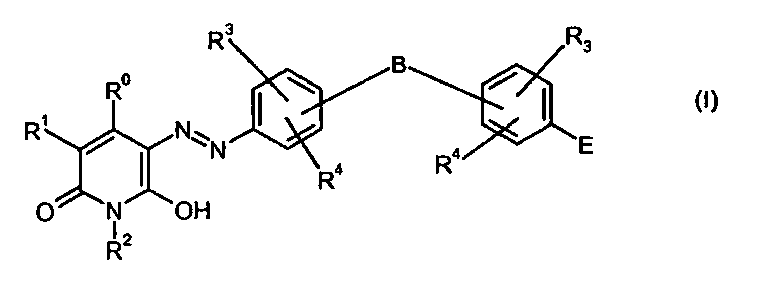 dans laquelle
E représente NH₂ ou OH,
R⁰ représente un groupe alkyle en C₁ à C₄ substitué ou un groupe alkyle en C₁ à C₄ non substitué,
R¹ représente H, un groupe alkyle en C₁ à C₄ substitué ou un groupe alkyle en C₁ à C₄ non substitué, un groupe sulfo, -CO-NH₂, -CO-NH-(alkyle en C₁ à C₄) ou CN,
R² représente H, un groupe alkyle en C₁ à C₄ substitué ou un groupe alkyle en C₁ à C₄ non substitué,
R³ représente H, un groupe sulfo, un groupe alkyle en C₁ à C₄ substitué ou un groupe alkyle en C₁ à C₄ non substitué, un groupe alcoxy en C₁ à C₄ substitué ou un groupe alcoxy en C₁ à C₄ non substitué,
R⁴ représente H, un groupe alkyle en C₁ à C₄ substitué ou un groupe alkyle en C₁ à C₄ non substitué, un groupe alcoxy en C₁ à C₄ substitué ou un groupe alcoxy en C₁ à C₄ non substitué,
B représente un groupe répondant à la formule -SO₂- -NH-CO-NH-, -CR⁵R⁶-, dans lequel
R⁵ représente H, un groupe alkyle en C₁ à C₉ substitué ou un groupe alkyle en C₁ à C₉ non substitué,
R⁶ représente H, un groupe alkyle en C₁ à C₉ substitué ou un groupe alkyle en C₁ à C₉ non substitué, un groupe aryle non substitué ou un groupe aryle substitué ou
R⁵ et R⁶ forment ensemble un cycle cycloaliphatique à cinq ou six chaînons, les cycles à cinq ou six chaînons étant substitués par un groupe alkyle en C₁ à C₄ ou les cycles à cinq ou six chaînons n'étant pas de plus substitués.

2. Composés selon la revendication 1 **caractérisés en ce que** les composés de formule (I) portent au moins un substituant anionique.

3. Composés selon la revendication 2 **caractérisés en ce que**
E représente NH₂ ou OH,
R⁰ représente un groupe alkyle en C₁ à C₂ non substitué,
R¹ représente un groupe alkyle en C₁ à C₂ substitué ou un groupe alkyle en C₁ à C₂ non substitué, un groupe sulfo, -CO-NH₂, -CO-NH- (alkyle en C₁ à C₂) ou CN,
R² représente un groupe alkyle en C₁ à C₃ substitué ou un groupe alkyle en C₁ à C₃ non substitué,
R³ représente H, un groupe sulfo, un groupe alkyle en C₁ à C₂ non substitué, un groupe alcoxy en C₁ à C₂ non substitué,
R⁴ représente H, un groupe alkyle en C₁ à C₂ non substitué, un groupe alcoxy en C₁ à C₂ non substitué,
B représente un groupe répondant à la formule -SO₂- -CR⁵R⁶-, dans lequel
R⁵ rebrprésente H, un groupe alkyle en C₁ à C₉ substitué ou un groupe alkyle en C₁ à C₉ non substitué,
R⁶ représente un groupe alkyle en C₁ à C₉ substitué ou un groupe alkyle en C₁ à C₉ non substitué, un groupe aryle non substitué ou un groupe aryle substitué ou
R⁵ et R⁶ forment ensemble un cycle cycloaliphatique à cinq ou six chaînons, les cycles à cinq ou six chaînons étant substitués par un groupe alkyle en C₁ à C₄ ou les cycles à cinq ou six chaînons n'étant pas de plus substitués.

4. Composés selon la revendication 3 **caractérisés en ce que**
E représente NH₂ ou OH,
R⁰ représente un groupe méthyle,
R¹ représente -CH₂-SO₃H, -CONH₂ ou -CN,
R² représente un groupe éthyle, -CH₂-CH₂-CH₂-NH-CH₃, -CH₂-CH₂-COOH ou -CH₂ -CH₂-CH₂-0-CH₂-CH₂-CH₂-CH₃,
R³ représente H, un groupe méthyle, méthoxy ou sulfo,
R⁴ représente H, un groupe méthyle ou méthoxy,
B représente un groupe répondant à la formule -SO₂- ou -CR⁵R⁶-, dans lequel
R⁵ représente H, un groupe méthyle ou éthyle,
R⁶ représente un groupe alkyle en C₁ à C₄ non substitué, un groupe aryle substitué ou
R⁵ et R⁶ forment ensemble un cycle cycloaliphatique à six chaînons, les cycles à six chaînons n'étant pas de plus substitués.

5. Procédé pour la préparation de composés représentés par la formule (I) selon la revendication 1, **caractérisé en ce que** les fonctions amines des composés représentés par la formule (II) 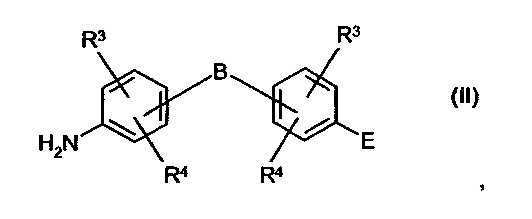 sont diazotées et couplées totalement à un équivalent d'un composé représenté par la formule (III) 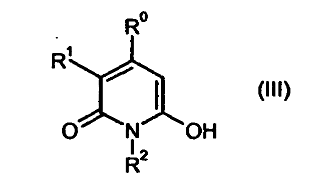 où les substituants sont chacun tels que définis ci-dessus.

6. Utilisation des composés représentés par la formule (I) selon la revendication 1 pour la teinture et/ou l'impression de substrats organiques.

7. Utilisation de composés de formule (I) selon la revendication 1 pour la teinture et/ou l'impression de laine, de soie et de polyamides synthétiques.

8. Utilisation de composés de formule (I) selon la revendication 1 pour la préparation d'encres d'impression pour le procédé par jet d'encre.
